# EUROPEAN PATENT APPLICATION

(11) **EP 4 101 393 A1**
(43) Date of publication of application: **14.12.2022**
(21) Application number: 21750437.2
(22) Date of filing: 03.02.2021
(51) Int. Cl.: A61B 10/00, A61B 10/02, A61B 10/04, A61B 1/018, G01N 33/543, G01N 33/569

(54) **METHOD FOR COLLECTING SAMPLE FROM GASTROINTENSTINAL MUCOUS MEMBRANE**

(30) Priority: 03.02.2020 JP 2020016181
(71) Applicant: NATIONAL UNIVERSITY CORPORATION HAMAMATSU UNIVERSITY SCHOOL OF MEDICINE, Hamamatsu-shi, Shizuoka 431-3192 (JP); Denka Company Limited, Tokyo 103-8338 (JP)
(72) Inventor: FURUTA Takahisa, Hamamatsu-shi, Shizuoka 431-3192 (JP); YAMADE Mihoko, Hamamatsu-shi, Shizuoka 431-3192 (JP); KAGAMI Takuma, Hamamatsu-shi, Shizuoka 431-3192 (JP); SUZUKI Takahiro, Hamamatsu-shi, Shizuoka 431-3192 (JP); HIGUCHI Tomohiro, Hamamatsu-shi, Shizuoka 431-3192 (JP); MIYAZAWA Takashi, Gosen-shi, Niigata 959-1695 (JP)
(74) Representative: Schrell, Andreas
(86) International application number: PCT/JP2021/003814
(87) International publication number: WO 2021/157586

(57) **Abstract**

Provided is a non-invasive and prompt testing method, in which a mucus adhering onto the surface of a mucous membrane is used as a specimen.

A method for collecting a specimen by wiping out a mucus adhering onto the surface of a mucous membrane, when the specimen is collected during an endoscopic examination.

## Description

### Technical Field

The present invention provides a method for collecting, as a specimen, a mucus adhering onto the surface of a mucous membrane from a body cavity, using an endoscope, and a testing method for detecting a substance to be detected in the specimen.

### Background Art

Conventionally, a method comprising observing a tubular organ, such as a blood vessel, a ureter, a bile duct or a pancreatic duct, or the body cavity of a human body, such as small intestine or large intestine, using an endoscope, and thereby examining the conditions of the biological tissues in such a tubular organ or a body cavity, has been broadly carried out. When a lesion has been diagnosed using an endoscope, an insertion section of the endoscope has been inserted into a body cavity, and the inner surface of a lumen has been observed according to endoscopic observation, so that the lesion site has been detected based on the morphological change thereof. After detection of the lesion site, a cell collecting means such as a cytology brush has been inserted into a treating tool channel of the endoscope, and while observing the lesion site, a mucous membrane at the lesion site has been scraped using the cytology brush, so as to collect cells. Thereafter, a diagnosis has been made from the results of microscopic observation of the collected cells, etc.

For example, for the definitive diagnosis of *Helicobacter pylori* gastritis, an endoscopic diagnosis is essentially carried out, and in general, the diagnosis of *Helicobacter pylori* infection is made after implementation of the endoscopic diagnosis. Along with this, gastric biopsy is carried out during the endoscopic examination, and also, RUT (Rapid urease test) as an invasive test, microscopy, or a culture method is carried out in many cases. However, gastric biopsy is problematic in that when a site that is not infected with *Helicobacter pylori* is collected upon collection of the tissues, false negative results come out, and also in that a patient who is administered with an antithrombotic drug such as aspirin or warfarin takes risks (Non Patent Literature 1). Hence, it has been desired to develop a method for testing *Helicobacter pylori,* which has low invasiveness and high accuracy, and enables prompt determination.

Other than low-invasive testing methods, namely, other than a urea breath test (UBT), a serum antibody measurement method and a stool antigen measurement method, there is a method of directly detecting *Helicobacter pylori* from a gastric fluid. It is relatively easy to collect a gastric fluid when an endoscopic diagnosis is carried out. Also, this is a non-invasive specimen collection method, which does not pose risks to patients. However, the amount of *Helicobacter pylori* cells in the collected gastric fluid is small, and thus, it is not easy to detect *Helicobacter pylori* with high sensitivity (Non Patent Literature 2). The culture method is disadvantageous in that the detection percentage according to this method is not high (0% to 67%), and also in that a considerable period of time is required for the detection. As a high-sensitive detection method, a PCR method has also been attempted. However, the PCR method is problematic in that a considerable period of time is required until determination results are obtained, in that risk management is required for contamination, in that special equipment is needed, and in that there are problems in terms of costs or operational complications. Accordingly, it is not easy to use the PCR method in actual medical sites.

### Citation List

### Non Patent Literature

Non Patent Literature 1: Guidelines for Gastrointestinal Endoscopy to Patients Administered with Antithrombotic Drugs; Digestive Endoscopy (2012)
Non Patent Literature 2: Med Microbiol. Vol. 49(4): 343-7, 2000

### Summary of Invention

### Technical Problem

The present invention provides a non-invasive and prompt testing method by using, as a specimen, a mucus adhering onto the surface of a mucous membrane.

### Solution to Problem

The inventors of the present application have conducted intensive studies. As a result, the present inventors have discovered that when a gastric mucus that can be collected during an endoscopic examination is used as a specimen, such a specimen can be easily collected by wiping out a mucus adhering onto the surface of a gastric mucous membrane using a swab, and that a measurement system, in which sufficient sensitivity can be obtained without being influenced by the pH of a gastric juice, is established, so that the usefulness of a scraped material from the gastric mucous membrane as a clinical specimen is found, thereby completing the present invention that enables prompt detection of *Helicobacter pylori* by non-invasive specimen collection.

The aspects of the present invention are as follows.
[1] A method for collecting a specimen by wiping out a mucus adhering onto the surface of a mucous membrane, when the specimen is collected during an endoscopic examination.
[2] The method according to the above [1], which is characterized in that the mucus adhering onto the surface of a mucous membrane is wiped out with a swab that is a treating tool of the endoscope.
[3] The method according to the above [1], which is characterized in that the mucus adhering onto the surface of a mucous membrane is wiped out with a cytology brush that is a treating tool of the endoscope.
[4] The method according to any one of the above [1] to [3], which is characterized in that the adhering mucus is wiped out from the surface of a gastric mucous membrane, using a treating tool of the endoscope.
[5] The method according to any one of the above [1] to [3], which is characterized in that the adhering mucus is wiped out from the surface of a mucous membrane of small intestine, using a treating tool of the endoscope.
[6] The method according to any one of the above [1] to [3], which is characterized in that the adhering mucus is wiped out from the surface of a mucous membrane of large intestine, using a treating tool of the endoscope.
[7] The method according to the above [4], which is characterized in that *Helicobacter pylori* is detected from the adhering mucus.
[8] A method for detecting *Helicobacter pylori* that is infected into a gastric mucous membrane, using, as a specimen, the mucus adhering onto the surface of the gastric mucous membrane, which is collected by the method according to any one of the above [1] to [7].
[9] The method according to the above [8], wherein an immunoassay is used in the detection.
[10] The method according to the above [8] or [9], wherein a *Helicobacter* pylori-specific antibody is used in the immunoassay.
[11] The method according to the above [9] or [10], wherein the immunoassay is an immunochromatography and/or an ELISA method.
[12] The method according to any one of the above [9] to [11], wherein the *Helicobacter pylori-*specific antibody is a polyclonal antibody and/or a monoclonal antibody that recognize(s) a structural factor and/or a pathogenesis factor of *Helicobacter pylori.*

The present description includes the contents as disclosed in Japanese Patent Application No. 2020-016181, which is a priority document of the present application.

### Advantageous Effects of Invention

According to the method of the present invention, a method of diagnosing *Helicobacter pylori* infection from the surface of a mucous membrane could be established.

### Description of Embodiments

Hereinafter, the present invention will be described in detail.

The present invention relates to a method for collecting a specimen by wiping out a mucus adhering onto the surface of a mucous membrane, when the specimen is collected during an endoscopic examination.

A method comprising observing a tubular organ, such as a blood vessel, a ureter, a bile duct or a pancreatic duct, or the body cavity of a human body, such as small intestine or large intestine, using an endoscope, and thereby examining the conditions of the biological tissues in such a tubular organ or a body cavity, has been broadly carried out. When a lesion is diagnosed using an endoscope, an insertion section of the endoscope is inserted into a body cavity, and the inner surface of a lumen is observed according to endoscopic observation. In order to wipe out a mucus of interest, a mucus membrane surface region is searched. A specimen-collecting tool for endoscope, such as a swab or a cytology brush, is inserted into a treating tool channel of the endoscope, and while observing the mucous membrane surface region, the surface of the mucous membrane is scraped to collect an adhering mucus. Thereafter, this specimen-collecting tool for endoscope is pulled out, so that the collection operations are terminated. Thereafter, the collected adhering mucus is used as a specimen, and various types of tests are carried out.

In order to achieve the aforementioned object, in the case of the specimen-collecting tool for endoscope according to the present invention, a specimen can also be collected by being scraped with a swab, a brush, a product having a mesh-like or sponge-like tip, or an absorbent made of a cloth or the like. Accordingly, the specimen-collecting tool for endoscope according to the present invention can be used in the scraping cytology of a tumor having a high risk of bleeding.

The measurement method, in which the present invention is used, will be described, while taking a method of measuring *Helicobacter pylori* as an example.

In the method of measuring *Helicobacter pylori, Helicobacter pylori* is measured according to an immunoassay of utilizing an antigen-antibody reaction between an anti*-Helicobacter* pylori-specific antibody or an antigen-binding fragment thereof and *Helicobacter pylori* in a specimen.

As such an *anti-Helicobacter pylori* antibody, either a monoclonal antibody or a polyclonal antibody can be used. The *anti-Helicobacter pylori* antibody used in the method of the present invention can be produced by a known method using, as an immunogen, a cell mass of *Helicobacter pylori* or a protein derived from *Helicobacter pylori.* The *anti-Helicobacter pylori* antibody used in the method of the present invention is an antibody specific to an antigen of *Helicobacter pylori.* Examples of the antigen of *Helicobacter pylori* may include: structural factors, such as flagellum and LPS; and pathogenesis factors, such as urease, adhesin, catalase, and proteins encoded by SOD, VacA, CagA and cagPAI gene clusters, OipA, NapA, DupA, and heat shock proteins. By detecting the antigen of *Helicobacter pylori,* the presence of *Helicobacter pylori* can be detected. Examples of the antigen-binding fragment of the *anti-Helicobacter pylori* antibody may include: immunoglobulin fragments that react with *Helicobacter pylori,* such as Fab and F(ab')2; and recombinant antibodies that are expressed as recombinants, such as scFv, dsFv, a diabody, and a minibody. In the present invention, the term "antibody" includes the aforementioned fragments that are specific to *Helicobacter pylori.* The method of preparing these fragments is publicly known in the present technical field.

Examples of the immunoassay method that can be used herein may include all methods that are publicly known to those skilled in the art, such as immunostaining methods (including a fluorescent antibody method, an enzyme antibody method, a heavy metal-labeled antibody method, and a radioisotope-labeled antibody method), methods of combining separation according to electrophoresis with a detection method using fluorescence, enzyme or radioisotope (including a Western blot method and fluorescent two-dimensional electrophoresis), an enzyme-linked immunosorbent assay (ELISA), a dot blotting method, a latex agglutination-turbidimetric immunoassay (LA), and immunochromatography. Besides, in the present invention, the "measurement" includes all of quantification, semi-quantification, and detection.

Among the aforementioned immunoassay methods, a sandwich method is preferable. The sandwich method itself is publicly known in the field of immunoassay, and the sandwich method can be carried out, for example, according to immunochromatography or an ELISA method, in which an immunoassay is performed in a lateral flow manner. All of these sandwich methods are publicly known, and the method of the present invention can be carried out according to a publicly known sandwich method, with the exception that the above-described *Helicobacter* pylori-specific monoclonal antibody and/or polyclonal antibody of the present invention are used.

In an immunoassay involving a sandwich method as a detection principle, as a solid phase on which the antibody is immobilized, all of those capable of immobilizing the antibody thereon according to a known technique can be used. For example, a known solid phase, such as a porous thin membrane (membrane) having capillary action, a particulate substance, a test tube, or a resin flat plate, can be arbitrarily selected. In addition, as a substance that labels the antibody, an enzyme, a radioisotope, a fluorescent substance, a luminescent substance, a colored particle, a colloidal particle, etc. can be used. In addition, two or more types of *anti-Helicobacter pylori* antibodies may also be used. Such two or more types of *anti-Helicobacter pylori* antibodies are preferably antibodies, which are used in the sandwich method and recognize each different epitopes.

For example, a stomach content sample is added onto a microtiter plate made of polystyrene or the like, on which an anti*-Helicobacter pylori* antibody is immobilized, followed by performing an antigen-antibody reaction, and thereafter, an enzyme-labeled anti*-Helicobacter pylori* antibody is further added thereto, followed by performing an antigen-antibody reaction. After the reaction mixture is washed, the resultant is allowed to react with an enzyme substrate and to develop color, and absorbance is then measured, so that the presence of *Helicobacter pylori* in the gastric fluid can be detected, and at the same time, the number of *Helicobacter pylori* cells in the gastric fluid can also be calculated from the obtained measurement value. Moreover, a fluorescently-labeled anti-*Helicobacter pylori* antibody may be subjected to an antigen-antibody reaction, and fluorescence may be then measured.

From the viewpoint of, in particular, the simplicity and rapidity of a clinical test, among the aforementioned immunoassay methods using various materials, immunochromatography, which is a lateral flow immunoassay method using a membrane, is preferable.

The lateral flow immunoassay method involving the method of the present invention can be carried out using an immunoassay instrument consisting of: a support having a detection region, on which an antibody (Antibody 1) for capturing a measurement subject (an antigen) is immobilized; a label region having a movable labeled antibody (Antibody 2) that is labeled with a suitable labeling substance such as colored polystyrene particles or gold colloids; a specimen pad for adding dropwise a specimen; an absorption band for absorbing a spread specimen solution, and a backing sheet for adhering these members to one another, in which at least either Antibody 1 or Antibody 2 is the *anti-Helicobacter pylori* antibody of the present invention. According to the present method, utilizing capillary action, a complex of: Antibody 2 capable of binding to a substance to be detected, which has been labeled with a suitable labeling substance such as a colored polystyrene particle or a gold colloid (a labeling reagent); and the substance to be detected, is developed and moved to a solid-phase support, on which Antibody 1 is immobilized. As a result, a complex consisting of an immobilized substance, a substance to be detected, and a labeling reagent is formed on the solid-phase support, and the signals of the labeling reagent emitted from the complex are then detected (in the case of using a gold colloid, the solid-phase support portion, on which the substance capable of binding to the substance to be detected is immobilized, becomes red), so that the substance to be detected can be detected. This immunoassay method can be carried out at a temperature of 5°C to 35°C, and preferably at room temperature. A biological mucous membrane-derived specimen may also be treated with a specimen-treating solution in this temperature range.

The present invention also includes the above-described immunoassay instrument for detecting *Helicobacter pylori* from a mucus adhering onto the surface of a gastric mucous membrane.

Besides, the number of detection regions and the type of labeled antibodies contained in the label region are not limited to 1. By using antibodies corresponding to a plurality of measurement subjects, two or more antigens can be detected using a single immunoassay instrument.

In the present invention, *Helicobacter pylori* is directly detected in a mucus adhering onto the surface of a gastric mucous membrane, so that *Helicobacter pylori* infected into the stomach can be detected. The mucus adhering onto the surface of a gastric mucous membrane is collected using a collecting tool such as an endoscopic swab or a cytology brush during endoscopic examination. Such a collecting tool is inserted into an endoscope thorough a treating tool channel (forceps port) of the endoscope, and is then discharged from a tip portion of the endoscope, so that a gastric mucous membrane can be collected from a desired site, while observing a gastric mucous membrane surface region by the endoscope. The mucus adhering onto the surface of a gastric mucous membrane may be collected, when the endoscope is inserted into the stomach. Otherwise, a stomach content may be previously removed from the stomach by aspiration or the like, and thereafter, a mucous membrane at a site in which the rubefaction or swelling of the mucous membrane possibly caused by *Helicobacter pylori* is found, or at a site suspected to be infected, may be collected using a collecting tool such as a swab. When the endoscope is inserted into the stomach, a defoaming agent is previously taken, or an anesthetic solution is injected into the nasal cavity or the throat. Thus, the stomach content is diluted with such a drug. Moreover, while the endoscope and the like scrape and stimulate the wall of the stomach, a gastric fluid is secreted and the stomach content is diluted with the gastric fluid. As a result, the sensitivity to detect *Helicobacter pylori* may be decreased by using such a diluted stomach content in some cases. According to the method of the present invention, however, since a specimen is directly collected from the mucous membrane, the specimen is not diluted, and thus, the sensitivity to detect *Helicobacter pylori* is not decreased. According to the method using an *anti-Helicobacter pylori* antibody of the present invention, the collected specimen can be directly used as a sample without being treated by concentration, culture operations, etc. Moreover, the collected specimen may be mixed with a buffer solution to prepare a sample. As such a buffer solution, for example, a phosphoric acid buffer solution can be used, and the buffer solution may comprise a surfactant such as Tween 20 or serum albumin. For instance, when the assay is carried out by immunochromatography that is a lateral flow immunoassay method using a membrane, a specimen collected with a cotton swab is suspended in a buffer solution, and is used as a specimen sample. Since the gastric fluid comprises a gastric mucus, the gastric fluid has been considered not to be suitable for immunoassay. However, according to the method of the present invention, immunoassay can be carried out without being affected with the gastric mucus.

In the present invention, the gastric fluid and gastric mucus of animals such as humans, dogs, and cats are used as subjects.

The method of the present invention is a method for detecting *Helicobacter pylori* infected into the stomach, and the present method is also a method for detecting the infection with *Helicobacter pylori,* or a method for collecting data used for detection of the infection with *Helicobacter pylori.*

When *Helicobacter pylori* is detected in the stomach and the infection with *Helicobacter pylori* is revealed according to the method of the present invention, the *Helicobacter pylori* infection can be treated by removing *Helicobacter pylori.* The removal of *Helicobacter pylori* can be carried out using antibiotics. At this time, the removal of *Helicobacter pylori* can be preferably carried out by the combined use of a plurality of antibiotics. For example, two antibiotic agents (i.e. amoxycillin (AMPC) and clarithromycin (CAM)) may be combined with a proton pump inhibitor (PPI) or a potassium-competitive acid blocker, which suppresses gastric-acid secretion, and may be then administered.

### Examples

Hereinafter, the present invention will be described in more detail, based on the following examples. However, the following examples are not intended to limit the scope of the present invention.

### 1. Production of anti-Helicobacter pylori monoclonal antibodies

BALB/c mice were immunized with antigens extracted from *Helicobacter pylori,* and were then bred for a certain period of time. Thereafter, the spleen was excised from each mouse, and was then fused with mouse myeloma cells (P3X63) according to the method of Kohler et al. (Kohler et al., Nature, vol, 256, pp. 495-497 (1975)). The obtained fusion cells (hybridomas) were maintained at 37°C in an incubator, and purification of the cells (monocloning) was then carried out, while the antibody activity in the supernatant was observed according to ELISA, using a plate on which the antigen extracted from *Helicobacter pylori* was immobilized. The obtained two cell lines were each administered intraperitoneally into pristane-treated BALB/c mice. Approximately 2 weeks after the administration, antibody-containing ascitic fluids were collected.

From each of the obtained ascitic fluids, IgG was purified by an affinity chromatography method using a Protein A column, and two types of purified anti-*Helicobacter pylori* antibodies were obtained.

### 2. Production of labeled anti-Helicobacter pylori antibody

One type of the anti*-Helicobacter pylori* antibody was dialyzed against a 50 mM MES (2-Morpholinoethanesulfonic acid, monohydrate; DOJINDO LABORATORIES) buffer (pH 6.0) solution, and was then diluted with the same buffer solution to result in O.D. 280 nm = 0.5, thereby preparing 10 mL of a diluted solution. Subsequently, the obtained solution was mixed with 10 (w/v)% blue polystyrene latex particles (particle diameter: 0.45 µm; surface functional groups: carboxyl groups; functional group density: 65Å2/COOH groups; Magsphere) at a liquid amount ratio of 40 : 1, followed by performing a reaction. Subsequently, 1 (w/v)% EDAC (N-(3-Dimethylaminopropyl)-N'-ethylcarbodiimide hydrochloride; Sigma) was added to the mixed solution to a final concentration of 0.1%, and the thus obtained mixture was reacted for 2 hours. After washing, the reaction mixture was suspended in 20 mL of a final suspension (5 mM Tris, 0.04 (w/v)% BSA (bovine serum albumin), 0.4 M trehalose, and 0.2 (v/v)% Triton X-100), and was then subjected to an ultrasonic disperser (Olympus Corporation), so that latex particles were dispersed. Thereafter, using a positive pressure spraying machine (BioJet; BioDot), the latex particle-labeled antibody was sprayed in an applied amount of 8 µL/cm onto the entire surface of a cellulose non-woven fabric having a width of 15 mm, which was wound on a reel. After completion of the spraying, hot air at 50°C was blown to the surface for 1 minute to dry it, so as to produce a latex particle-labeled antibody dry pad.

### 3. Preparation of antibody to be immobilized on membrane

The purified anti*-Helicobacter pylori* antibody produced in the above 1., which had not been used in labeling, was dialyzed against a solid phase solution (10 mM Tris-HCl (pH 8.0)), and after completion of the dialysis, it was filtered through a 0.22 µm filter. The resultant was diluted with the solid phase solution to result in O.D. 280 nm = 3.0, thereby preparing an *anti-Helicobacter pylori* antibody to be immobilized.

### 4. Production of assay device for detection of Helicobacter pylori

As a membrane, a sheet (white) of a nitrocellulose membrane (pore diameter: 12 µm; manufactured by WATT MANN CO., LTD.) with a size of width 3 cm x length 10 cm was used. An anti*-Helicobacter pylori* antibody to be immobilized was linearly applied in an applied amount of 1 µL/cm onto a position 6 mm apart from an end on the long axis side of the sheet (wherein this end is referred to as an "upstream end" and the opposite end is referred to as a "downstream end"), using a positive pressure spraying machine (BioJet; BioDot). On the other hand, an anti-mouse IgG antibody diluted to O.D. 280nm = 1.0 was linearly applied in an applied amount of 1 µL/cm onto a position 22 mm apart from the one end of the long axis side, using a positive pressure spraying machine (BioJet; BioDot). After completion of the application, hot air at 45°C was blown to each of the applied antibodies for 10 minutes, so as to dry the antibodies.

Subsequently, in order to fix the member and increase the strength thereof, a backing sheet made of plastic (manufactured by BioDot) was adhered to the surface (which is referred to as a "lower surface") opposite to the antibody-applied surface of the membrane (wherein this surface is referred to as an "upper surface").

Subsequently, the latex particle-labeled antibody dry pad produced in the above 2. was cut into a section with width 15 mm x length 10 cm, and the section was then disposed and adhered onto the upper surface of the membrane, such that the upstream end of the membrane could be 2 mm overlapped with the section. Further, a cellulose filter (WATT MANN CO., LTD.) with a size of width 23 mm x length 10 cm was disposed and adhered onto the upper surface of the latex particle-labeled antibody dry pad, such that the filter could be 13 mm overlapped with the pad, so as to prepare a sample dropping pad.

Thereafter, a cellulose filter (WATT MANN CO., LTD.) with a size of width 30 mm x length 10 cm was disposed and adhered onto the upper surface of the membrane, such that the filter could be 5 mm overlapped with the downstream end of the membrane, so as to prepare a sample absorbing pad.

Subsequently, the entire upper surface, excluding a width of 5 mm at the upstream end of the sample dropping pad, was covered with a transparent plastic laminate (Adhesive Research).

Finally, the resulting pad was cut by 5 mm each along the long axis direction, so as to produce a membrane assay device.

### 5. Detection of Helicobacter pylori

The specimen was subjected to the diagnosis of *Helicobacter pylori* infection according to a rapid urease test (RUT) and/or a PCR method. With regard to patients (10 patients) diagnosed to be positive (+) to *Helicobacter pylori* infection according to comprehensive findings and patients (3 patients) diagnosed to be negative (-) to *Helicobacter pylori* infection according to comprehensive findings, a gastric fluid collected by aspiration from the stomach of each patient and a swab fluid obtained by wiping the wall of the stomach with a swab during endoscopic examination were used.

Regarding a gastric fluid specimen, 500 µL of the gastric fluid was directly added into 1 mL of a specimen suspending buffer solution (a phosphoric acid buffer solution (pH 7.4) containing 0.05(w/v)% Tween 20 and 0.1(w/v)% bovine serum albumin), and the obtained mixture was then stirred, and the thus obtained reaction mixture was used as a specimen sample.

Regarding a swab fluid specimen, a swab that had been used to collect the specimen was immersed in 1 mL of a specimen suspending buffer solution (a phosphoric acid buffer solution (pH 7.4) containing 0.05 (w/v)% Tween 20 and 0.1 (w/v)% bovine serum albumin), and thereafter, a matter adhering to the tip was squeezed out and was then extracted in the specimen suspending buffer solution, which was then used as a specimen sample.

The sample dropping pad side of the lateral flow membrane assay device for use in detection of *Helicobacter pylori,* which had been produced in the above 4., was immersed in the specimen sample solution. Ten minutes later, the assay device was observed. When coloration was observed at a position onto which the anti-mouse IgG antibody had been applied (control line), it was determined to be valid. When coloration was observed at a position onto which the anti-*Helicobacter pylori* antibody to be immobilized had been applied, it was determined to be positive (+) to *Helicobacter pylori.* When coloration was not observed at a position onto which the *anti-Helicobacter pylori* antibody to be immobilized had been applied, it was determined to be negative (-) to *Helicobacter pylori.* Moreover, when coloration was not observed at the position of the control line, it was determined to be invalid.

### 6. Comparative studies

From the results shown in Table 1, it was found that the gastric fluid specimen exhibited false negative in the case of Specimen No.3, but that the results were improved in the case of the swab fluid specimen.

**[Table 1]**

| Specimen No. | Lateral flow membrane assay device | | RUT | PCR |
|---|---|---|---|---|
| | Gastric fluid specimen | Cotton swab fluid | | |
| No.1 | + | + | + | + |
| No.2 | + | + | + | + |
| No.3 | - | + | + | ND |
| No.4 | - | - | ND | - |
| No.5 | + | + | ND | + |
| No.6 | - | - | - | ND |
| No.7 | + | + | + | ND |
| No.8 | + | + | + | ND |
| No.9 | - | - | - | ND |
| No.10 | + | + | + | ND |
| No.11 | + | + | + | ND |
| No.12 | + | + | + | ND |
| No.13 | + | + | + | ND |

| | | | | |
|---|---|---|---|---|
| ND:No Data | | | | |

### Industrial Applicability

The present invention can be utilized in detection of infection with *Helicobacter pylori.*

All publications, patents and patent applications cited in the present description are incorporated herein by reference in their entirety.

## Claims

1. A method for collecting a specimen by wiping out a mucus adhering onto the surface of a mucous membrane, when the specimen is collected during an endoscopic examination.

2. The method according to claim 1, which is **characterized in that** the mucus adhering onto the surface of a mucous membrane is wiped out with a swab that is a treating tool of the endoscope.

3. The method according to claim 1, which is **characterized in that** the mucus adhering onto the surface of a mucous membrane is wiped out with a cytology brush that is a treating tool of the endoscope.

4. The method according to any one of claims 1 to 3, which is **characterized in that** the adhering mucus is wiped out from the surface of a gastric mucous membrane, using a treating tool of the endoscope.

5. The method according to any one of claims 1 to 3, which is **characterized in that** the adhering mucus is wiped out from the surface of a mucous membrane of small intestine, using a treating tool of the endoscope.

6. The method according to any one of claims 1 to 3, which is **characterized in that** the adhering mucus is wiped out from the surface of a mucous membrane of large intestine, using a treating tool of the endoscope.

7. The method according to claim 4, which is **characterized in that** *Helicobacter pylori* is detected from the adhering mucus.

8. A method for detecting *Helicobacter pylori* that is infected into a gastric mucous membrane, using, as a specimen, the mucus adhering onto the surface of the gastric mucous membrane, which is collected by the method according to any one of claims 1 to 7.

9. The method according to claim 8, wherein an immunoassay is used in the detection.

10. The method according to claim 8 or 9, wherein a *Helicobacter pylori-*specific antibody is used in the immunoassay.

11. The method according to claim 9 or 10, wherein the immunoassay is an immunochromatography and/or an ELISA method.

12. The method according to any one of claims 9 to 11, wherein the *Helicobacter pylori-*specific antibody is a polyclonal antibody and/or a monoclonal antibody that recognize(s) a structural factor and/or a pathogenesis factor of *Helicobacter pylori.*
